# EUROPÄISCHE PATENTANMELDUNG

(11) **EP 4 116 364 A1**
(43) Veröffentlichungstag der Anmeldung: **11.01.2023**
(21) Anmeldenummer: 21183712.5
(22) Anmeldetag: 05.07.2021
(51) Int. Cl.: C08J 11/22, C08G 69/26, C08L 77/06

(54) **VERFAHREN ZUR HERSTELLUNG VON MONOMEREN UND/ODER OLIGOMEREN AUS EINEM POLYMER**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung von Monomeren und/oder Oligomeren aus einem eine Struktureinheit mit einer Stickstoff-Carbonylkohlenstoff-Bindung umfassenden Polymer, wobei die Stickstoff-Carbonylkohlenstoff-Bindung in einer chemischen Reaktion mit Formaldehyd oder Paraformaldehyd als aktivierendes Reagenz gespalten wird und wobei die Reaktion unter Einsatz einer als Katalysator agierenden Lewis-Säure erfolgt. Die Erfindung betrifft weiter Monomere, insbesondere ein Carbonsäure-Monomer und ein Aldimin-Monomer oder ein Amin-Monomer, erhältlich oder hergestellt aus diesem Verfahren.

## Beschreibung

Die vorliegende Erfindung betrifft ein Verfahren zur Herstellung von Monomeren und/oder Oligomeren aus einem eine Struktureinheit mit einer Stickstoff-Carbonylkohlenstoff-Bindung umfassenden Polymer.

Die Erfindung betrifft zudem Monomere, insbesondere ein Carbonsäure-Monomer und ein Aldimin-Monomer oder ein Amin-Monomer, erhältlich oder hergestellt aus diesem Verfahren.

Es existieren eine Vielzahl synthetischer Polymere (u.a. Kunststoffe), die in unterschiedlichsten Bereichen eingesetzt werden, beispielsweise in der Verpackungs-, Automobil- und Elektronikindustrie, aber auch im Bauwesen und in der Medizin. Kunststoffe zeichnen sich einerseits durch ihre mannigfaltigen mechanischen, physikalischen und chemischen Eigenschaften aus, andererseits durch ihre relativ geringen Herstellungskosten. Als Alternative zu Neusynthesen besteht ein wachsendes Interesse an Recyclingtechnologien, die einerseits eine Wiederverwertung der Kunststoffe ermöglichen und andererseits wirtschaftlich konkurrenzfähig sind den Neusynthesen.

Eines der industriell bedeutsamsten synthetischen Polymere ist Polyamid. Bei Polyamiden handelt es sich um Polymere mit sich regelmäßig wiederholenden Amid-Bindungen entlang einer Polymer-Hauptkette. Solche Amid-Bindungen können durch Kondensation einer Carbonsäure und eines Amins gebildet werden, wobei eine Stickstoff-Carbonylkohlenstoff-Bindung ausgebildet wird. Je nach Anzahl der miteinander verknüpften und das Polymer ausbildenden Monomere kann ein Polymer eine Vielzahl von Struktureinheiten mit solchen Stickstoff-Carbonylkohlenstoff-Bindungen aufweisen. Als Monomere zur Herstellung von Polyamiden werden u.a. Aminocarbonsäuren, Lactame, Diamine und/oder Dicarbonsäuren verwendet. Die zwei am häufigsten verwendeten Polyamide sind Polyamid 6.6 und Polyamid 6 (auch bekannt als Nylon 6.6 bzw. Nylon 6). Polyamid 6.6 wird aus Hexamethylendiamin und Adipinsäure hergestellt. Es entsteht durch eine Polykondensation unter Wasserabspaltung. Polyamid 6 wiederum entsteht durch Ringöffnungspolymerisation aus ε-Caprolactam mit Wasser als Starter. Aufgrund ihrer Festigkeit und Zähigkeit werden Polyamide oft als Konstruktionswerkstoffe verwendet.

Ein anderes, ebenfalls industriell bedeutendes synthetisches Polymer ist Polyurethan. Polyurethane weisen als Charakteristikum sich regelmäßig wiederholende Urethan-Gruppen auf. Teil dieser Urethan-Gruppen ist - wie auch bei Amid-Bindungen - eine Stickstoff-Carbonylkohlenstoff-Bindung, wobei Urethan-Gruppen im Gegensatz zu Amid-Gruppen ein zusätzliches SauerstoffAtom aufweisen. Polyurethane entstehen bei der Polyadditionsreaktion von Polyolen mit Polyisocyanaten. Die Umsetzung von Dialkoholen (auch Diole genannt) mit Diisocyanaten führt zu linearen Polyurethanen, während die Reaktion von beispielsweise Triisocyanat-Diisocyanat-Gemischen mit Triol-Diol-Gemischen zu vernetzten Polyurethanen führt. Je nach Vernetzungsgrad und eingesetzter Isocyanat- und Alkohol-Komponente ergeben sich Polymere mit unterschiedlichen Eigenschaften. So können Polyurethane Duroplaste, Thermoplaste oder Elastomere bilden. Am häufigsten genutzt werden Polyurethane für die Herstellung von weichen oder harten Schaumstoffen. Sie können aber auch als pressbare Formmassen, als Gießharze (Isocyanat-Harze), als (textile) elastische Faserstoffe, als Lacke und als Klebstoffe verwendet werden.

Wie schon eingangs erwähnt, besteht ein großes Interesse daran, synthetische Polymere zu recyclen und insbesondere derart mittels chemischer Recyclingverfahren zu behandeln, dass die bei der Synthese eingesetzten Monomere oder zumindest Oligomere sowie niedermolekulare chemische Verbindungen zurückgewonnen werden können. Aus diesen Monomeren/Oligomeren können dann idealerweise wieder neue Polymere synthetisiert werden.

Derartige chemische Recyclingverfahren kommen sowohl bei Polyamiden als auch bei Polyurethanen zum Einsatz. Um die Polymerstrukturen zu brechen, werden - je nach zu recycelndem Polymer - unterschiedliche Verfahren eingesetzt.

Die EP 1 801 101 A1 offenbart beispielsweise ein Verfahren zur Herstellung von Monomeren aus einem eine Struktureinheit mit einer Stickstoff-Carbonylkohlenstoff-Bindung umfassenden Polymer. Offenbart ist ein Verfahren zur Herstellung von Monomeren aus Polyamiden. Bei dem Verfahren wird die Stickstoff-Carbonylkohlenstoff-Bindung in einer chemischen Reaktion mit einem Alkohol, der zwei oder mehr Kohlenstoff-Atome aufweist, als aktivierendes Reagenz gespalten. Die Reaktion erfordert keinen Katalysator und wird bei einer Reaktionstemperatur von ≥ 350 °C durchgeführt. Das Verfahren eignet sich insbesondere für Polyamid 6, aus dem mittels des Verfahrens je nach verwendetem Alkohol bis zu 97 % des Monomers ε-Caprolactam zurückgewonnen werden.

Auch aus der US 5,668,277 A ist ein Verfahren zur Herstellung von Monomeren aus Polyamid 6 bekannt. Bei dem Verfahren wird Polyamid 6 mit Hilfe von Ammoniak, einem Amin oder einer Mischung daraus als aktivierendem Reagenz gespalten. Die Reaktion findet bei einer Reaktionstemperatur zwischen 200 und 400 °C und einem Druck von etwa 0,5 bis 5 atm statt. Als Reaktionsprodukte entstehen unter anderem ε-Caprolactam und ε-Caprolactam-Vorläufermoleküle wie ε-Aminocapronsäure, die unmittelbar für die Neusynthese von Polyamiden genutzt werden können.

Für Polyamid 6.6 ist aus der US 4,605,762 A ein aufwendiges Verfahren bekannt, bei dem die Monomere durch Hydrolyse bei einer Reaktionstemperatur von 200 bis 300 °C und einem Druck von mindestens 15 atm aus dem Polyamid 6.6 zurückgewonnen werden.

Die wissenschaftliche Publikation "A new approach to chemical recycling of polyamide 6.6 and synthesis of polyurethanes with recovered intermediates" von Datta et al., J Polym Environ 26, 4415-4429 (2018), offenbart ein weiteres Verfahren zur Depolymerisation von Polyamid 6.6. Dabei wird Polyamid 6.6 mit Ethylenglykol oder einer Mischung als Ethylenglykol und Triethylentetramin als aktivierendes Reagenz in Präsenz eines Katalysators umgesetzt. Es entsteht eine Mischung von niedermolekularen Verbindungen, die unter anderem Hexamethylendiamin, eine Substanz mit endständigen β-Hydroxyethylester-Gruppen, Ester und Amine umfasst. Die Reaktionsprodukte konnten erfolgreich für die Neusynthese von Polyurethanen verwendet werden.

Auch wenn bereits Verfahren zur Depolymerisation synthetischer Polymere bekannt sind, die die Rückgewinnung von Monomeren und/oder Oligomeren umfassen, besteht weiterhin ein großer Bedarf an alternativen, weniger aufwendigen, umweltfreundlichen und kostengünstigen Verfahren zur Herstellung von Monomeren und/oder Oligomeren aus Polymeren.

Es ist daher die Aufgabe der vorliegenden Erfindung, ein Verfahren zur Herstellung von Monomeren und/oder Oligomeren aus einem eine Struktureinheit mit einer Stickstoff-Carbonylkohlenstoff-Bindung umfassenden Polymer anzugeben, das ein weniger aufwendiges, umweltfreundlicheres und kostengünstigeres Polymerrecycling erlaubt.

Diese Aufgabe wird gelöst durch ein Verfahren zur Herstellung von Monomeren und/oder Oligomeren aus einem eine Struktureinheit mit einer Stickstoff-Carbonylkohlenstoff-Bindung umfassenden Polymer, wobei die Stickstoff-Carbonylkohlenstoff-Bindung in einer chemischen Reaktion mit Formaldehyd oder Paraformaldehyd als aktivierendes Reagenz gespalten wird und wobei die Reaktion unter Einsatz einer als Katalysator agierenden Lewis-Säure erfolgt.

Ferner liegt der vorliegenden Erfindung die Aufgabe zugrunde, Monomere, insbesondere ein Carbonsäure-Monomer und ein Aldimin-Monomer oder ein Amin-Monomer, im Wege eines Recyclingverfahrens aus Polymeren mit im Vergleich zum Stand der Technik geringerem Aufwand, auf umweltfreundlichere Art und Weise sowie kostengünstiger herzustellen.

Diese Aufgabe wird gelöst durch Monomere, insbesondere ein Carbonsäure-Monomer und ein Aldimin-Monomer oder ein Amin-Monomer, die aus einem solchen Verfahren erhältlich oder hergestellt sind.

Es ist darauf hinzuweisen, dass die in den Ansprüchen einzeln aufgeführten Merkmale in beliebiger, technisch sinnvoller Weise miteinander kombiniert werden können (auch über Kategoriegrenzen, beispielsweise zwischen Verfahren und Vorrichtung, hinweg) und weitere Ausgestaltungen der Erfindung aufzeigen. Die Beschreibung charakterisiert und spezifiziert die Erfindung zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Bevorzugte Ausgestaltungen der Erfindung sind Gegenstand der abhängigen Ansprüche.

Erfindungsgemäß ist vorgesehen, dass aus einem Polymer, das eine Struktureinheit mit einer Stickstoff-Carbonylkohlenstoff-Bindung aufweist, Monomere und/oder Oligomere hergestellt werden, indem das Polymer in einer chemischen Reaktion mit Formaldehyd oder Paraformaldehyd als aktivierendes Reagenz umgesetzt wird. Dabei dient eine Lewis-Säure als Katalysator. Die während des Verfahrens stattfindende chemische Reaktion führt dazu, dass die Bindung zwischen dem Stickstoff-Atom und dem Carbonylkohlenstoff-Atom der Stickstoff-Carbonylkohlenstoff-Bindung gespalten wird, sodass in der Folge Monomere und/oder Oligomere aus den für das Verfahren eingesetzten Polymeren zurückgewonnen werden können. Der Terminus der "Struktureinheit" bezieht sich auf funktionelle Gruppen, welche insbesondere die im Polymer vorliegenden Wiederholungseinheiten miteinander verknüpfen.

Unter einer "Lewis-Säure" sei im Kontext der vorliegenden Erfindung ein elektrophiler Elektronenpaarakzeptor zu verstehen, der in der Lage ist, Elektronenpaare anzulagern. Zu den Lewis-Säuren zählen unter anderem Protonen und Metallkationen.

Als Reaktionsedukt (also als Ausgangssubstanz) für das erfindungsgemäße Verfahren eignen sich insbesondere synthetische Polymere. Besonders gut können aliphatische Polyamide oder aliphatische Polyurethane verwendet werden. Grundsätzlich ist vorstellbar, das erfindungsgemäße Verfahren bei sortenreinen Polymerabfällen einzusetzen, welche die Reaktionsedukte bereitstellen können. Möglich erscheint aber auch, das erfindungsgemäße Verfahren bei Abfallgemischen unterschiedlicher Polymere einzusetzen, allerdings mit der Notwendigkeit nachträglicher Aufreinigung und mit einer höheren Wahrscheinlichkeit für ungewünschte Nebenreaktionen. In der Regel wird ein Polymerabfallgemisch also vor Einsatz als Reaktionsedukt im hiesigen Verfahren getrennt bzw. aufgereinigt werden, sodass möglichst sortenreine Polymere als Reaktionsedukte vorliegen. Die Trennung von Polymerabfällen kann mechanisch, physikalisch oder chemisch erfolgen, d.h. unter Einsatz geeigneter Sortier- und Reinigungsverfahren.

Ein wesentlicher Vorteil des erfindungsgemäßen Verfahrens ist, dass es sich um eine weniger aufwendige, umweltfreundliche, kostengünstige, und effiziente Alternative zu den bereits aus dem Stand der Technik bekannten Verfahren zur Herstellung von Monomeren und/oder Oligomeren aus einem eine Struktureinheit mit einer Stickstoff-Carbonylkohlenstoff-Bindung umfassenden Polymer handelt.

Bei dem Verfahren kann Formaldehyd oder Paraformaldehyd stöchiometrisch eingesetzt werden. Unter dem Begriff "stöchiometrisch" sei hier zu verstehen, dass die Menge an eingesetztem Formaldehyd oder der eingesetzten Wiederholungseinheiten von Paraformaldehyd mit der eingesetzten Menge an Wiederholungseinheiten des Polymers identisch ist oder ein ganzes Vielfaches von letzterer beträgt. Durch den stöchiometrischen Einsatz von Formaldehyd oder Paraformaldehyd ist sichergestellt, dass für sämtliche Stickstoff-Carbonylkohlenstoff-Bindungen im Polymer mindestens ein Formaldehyd-Molekül bzw. Formaldehyd-Rest zur Verfügung steht, welches bzw. welcher die Bindung zwischen dem Stickstoff-Atom und dem Carbonylkohlenstoff-Atom der Stickstoff-Carbonylkohlenstoff-Bindung spalten kann. Somit ist zu erwarten, dass bei stöchiometrischem Einsatz von Formaldehyd oder Paraformaldehyd die Ausbeute an vom Polymer abgespaltenen Monomeren und/oder Oligomeren maximiert ist.

Alternativ kann Formaldehyd oder Paraformaldehyd auch substöchiometrisch eingesetzt werden. "Substöchiometrisch" wiederum bedeutet, dass die Menge an eingesetztem Formaldehyd oder der eingesetzten Wiederholungseinheiten von Paraformaldehyd nur einen Bruchteil der eingesetzten Menge an Wiederholungseinheiten des Polymers beträgt. Dies birgt den Vorteil, dass auf diese Weise bei der Durchführung des Verfahrens eine geringere Menge von aktivierendem Reagenz benötigt wird und somit Kosten eingespart werden können. Ferner ist ein geringerer Stoffeinsatz mit einer verbesserten Umweltbilanz assoziiert.

Als Lewis-Säure kann bevorzugt ein Metallkatalysator eingesetzt werden. Besonders bevorzugt kann der Metallkatalysator ein Metall aus der folgenden Gruppe umfassen: Aluminium, Bohr, Bismut, Cer, Eisen, Kupfer, Lanthan, Magnesium, Zinn, Titan, Zirkonium oder Zink. Die Metalle können vorzugsweise in Form ihrer Chlorid-Salze eingesetzt werden. Auch der Einsatz anderer Salze ist denkbar. Bevorzugte Oxidationsstufen der vorgenannten Metallkatalysatoren sind Aluminium-(III), Bohr-(III), Bismut-(III), Cer-(III), Eisen-(III), Kupfer-(II), Lanthan-(III), Magnesium-(II), Zinn-(IV), Titan-(IV), Zirkonium-(IV) oder Zink-(II).

Gemäß einer weiteren Ausgestaltung der Erfindung kann als Metallkatalysator eine Metall-Triflat-Verbindung, vorzugsweise Bismut-Triflat, eingesetzt werden. Triflat-Verbindungen wurden in den letzten Jahren verstärkt erfolgreich als Lewis-Säuren in vielen Reaktionen eingesetzt. Der Vorteil dieser Salz-Verbindungen ist die extreme Stabilität des Triflat-Anions. Selbst in Wasser gelöst reagiert das Triflat-Anion im Gegensatz zu vielen anderen klassischen Lewis-Säuren nicht weiter. Dies führt dazu, dass die bei der Durchführung des Verfahrens stattfindende chemische Reaktion besser kontrolliert und unerwünschte Nebenreaktionen besser vermieden werden können.

Neben Bismut-Triflat eignen sich beispielswiese Scandium-Triflat, Neodym-Triflat, Samarium-Triflat, Yttrium-Triflat, Lanthan-Triflat oder Gadolinium-Triflat als Metall-Triflat-Verbindung.

Ferner kann die chemische Reaktion in einem organischen Lösungsmittel, insbesondere in einem polaren organischen Lösungsmittel, durchgeführt werden. Bei Benötigung eines Lösungsmittels, z.B. weil das eingesetzte Polymer, das aktivierende Reagenz und/oder der Katalysator in fester Phase vorliegen, dient ein organisches Lösungsmittel dazu, die an der Reaktion beteiligten Moleküle in besseren Kontakt zueinander zu bringen, indem eine oder mehrere der eingesetzten Verbindungen ganz oder zumindest teilweise gelöst werden. Die Verwendung eines polaren organischen Lösungsmittels hat den Vorteil, dass sich Metall-Triflat-Verbindungen, wie sie bei der Erfindung als katalytisch aktive Lewis-Säure zum Einsatz kommen können, gut darin lösen. Welche (polaren) organischen Lösungsmittel für das Verfahren eingesetzt werden können, hängt stark von dem eingesetzten Polymer, dem aktivierenden Reagenz und dem gewählten Katalysator ab. Als polare organische Lösungsmittel eignen sich beispielsweise Dioxolan, Tetrahydrofuran, Dioxan, Dimethoxyethan, Wasser, o-Dichlrobenzol, Dimethylsulfoxid, Dimethylformamid, Methanol, Ethanol, Ethylenglykol, Essigsäure, Trioxan oder Chloroform, bevorzugt Trioxan oder Chloroform.

Darüber hinaus kann für das Verfahren Wasser als Additiv eingesetzt werden. Der Einsatz von Wasser als Additiv führt dazu, dass das Reaktionsgleichgewicht der bei der Durchführung des Verfahrens stattfindenden chemischen Reaktion zugunsten der Reaktionsprodukte verschoben wird. Denn Wasser fördert die Spaltung von Stickstoff-Carbonylkohlenstoff-Bindungen und somit die Bildung weiterer Monomere und/oder Oligomere. Wird zusätzlich zum additiven Wasser eine Metall-Triflat-Verbindung als Katalysator verwendet, bleibt das bei der Zugabe von Wasser entstehende Triflat-Anion in der Lösung stabil und reagiert vorteilhafterweise nicht weiter. Das Wasser kann auch kontinuierlich zugegeben werden, um die Hydrolyse zu verstärken ohne die Lewis-Säure zu stark zu dämpfen.

Nach einer weiteren Ausgestaltung der Erfindung kann die Herstellung der Monomere und/oder Oligomere in einer eine erste und eine zweite Reaktionsstufe umfassenden Reaktion erfolgen. Dabei kann die erste Reaktionsstufe bei einer ersten Reaktionstemperatur von ≤ 100 °C, bevorzugt ≤ 80 °C, besonders bevorzugt ≤ 60 °C erfolgen. Die zweite Reaktionsstufe wiederum kann bei einer zweiten Reaktionstemperatur von ≤ 200 °C, bevorzugt ≤ 180 °C, besonders bevorzugt ≤ 165 °C erfolgen. Alternativ können die erste und die zweite Reaktionsstufe beide bei einer einheitlichen Reaktionstemperatur, idealerweise bei der zweiten Reaktionstemperatur, also bei ≤ 200 °C, bevorzugt bei ≤ 180 °C, besonders bevorzugt bei ≤ 165 °C, erfolgen. Es hat sich herausgestellt, dass die erste Reaktionsstufe bereits bei einer niedrigeren Reaktionstemperatur stattfindet. Die erste und die zweite Reaktionsstufe können aber bei derselben Reaktionstemperatur durchgeführt werden, wodurch sich die Durchführung des Verfahrens vereinfacht, da die Reaktionstemperatur nur einmal eingestellt werden muss. Im Vergleich zu anderen, aus dem Stand der Technik bekannten Verfahren zur Herstellung von Monomeren und/oder Oligomeren aus Polymeren sind die hier verwendeten Reaktionstemperaturen vergleichsweise niedrig (milde Reaktionsbedingungen). Demnach ist das hier beschriebene Verfahren energieeffizienter und umweltfreundlicher als andere (chemische) Recyclingverfahren für Polymere.

In der ersten Reaktionsstufe kann ein Halbaminal gebildet werden. Dies ist insbesondere der Fall, wenn als Polymer ein Polyamid oder ein Polyurethan eingesetzt werden.

In der zweiten Reaktionsstufe können wiederum eine Carbonsäure und ein Aldimin gebildet werden. Dies ist dann der Fall, wenn als Polymer ein Polyamid eingesetzt wird. Bei einer Carbonsäure und einem Aldimin handelt es sich um besonders einfache Strukturen, die gut für neue Synthesereaktionen eingesetzt werden können.

Anschließend kann das Aldimin weiter umgesetzt werden zu einem Amin. Bei einem Amin handelt es sich im Vergleich zum Aldimin um eine noch simplere Struktur, die sich ebenfalls bestens für die Wiederverwertung in neuen Synthesereaktionen eignet.

Mit dem Verfahren können aliphatische, araliphatische oder cycloaliphatische Amine hergestellt werden. Das hergestellte Amin kann insbesondere ein Amin aus der folgenden Gruppe umfassen: Tetramethylendiamin, Pentamethylendiamin (PDA), Hexamethylendiamin (HDA), 2-Methyl-1,5-diaminopentan, 1,5-Diamino-2,2-dimethyl-pentan, 2,2,4- bzw. 2,4,4-Trimethyl-1,6-diaminohexan, 1,10-Diaminodecan, 1,3- und 1,4-Diaminocyclohexan, 1,3- und 1,4-Bis-(aminomethyl)-cyclohexan (H6-XDA), 1-Isocyanato-3,3,5-trimethyl-5-isocyanatomethylcyclohexan (Isophorondiamin; IPDA), 2,4'- und 4,4'-Diaminodicyclohexylmethan, 1,3- und 1,4-Bis(aminomethyl)benzol (XDA) oder Bis(aminomethyl)norbornan (NBDA). Besonders bevorzugt umfasst das hergestellte Amin ein Amin aus der folgenden Gruppe: Pentamethylendiamin (PDA), Hexamethylendiamin (HDA), Isophorondiamin (IPDA), 2,4'- und 4,4'-Diaminodicyclohexylmethan, 1,3- und 1,4-Diaminocyclohexan, 1,3- und 1,4-Bis-(aminomethyl)-cyclohexan (H6-XDA), 1,3- und 1,4-Bis(aminomethyl)benzol (XDA) oder Bis(aminomethyl)norbornan (NBDA). Bedingung für die Herstellung der genannten Amine ist, dass die eingesetzten Polymere entsprechende Wiederholungseinheiten aufweisen, die eine Synthese der genannten Amine erlauben.

Alternativ oder zusätzlich können mit dem Verfahren Alkohole und Isocyanate hergestellt werden. Dabei kann es sich sowohl um Monomere als auch um Oligomere handeln. Alkohol und Isocyanat können dann gebildet werden, wenn als Polymer ein Polyurethan-Polymer eingesetzt wird. Auch Alkohole und Isocyanate sind einfache Strukturen, die sich gut für Neusynthesen eignen. Ein Beispiel für ein mögliches Reaktionsprodukt, wenn ein entsprechendes Polyurethan als Polymer eingesetzt wird, ist 4,4'-Diisocyanatodicyclohexylmethan.

Weitere Einzelheiten und Vorteile der Erfindung ergeben sich aus den nachfolgend beschriebenen Modellreaktionen und einem Ausführungsbeispiel.

Alle eingesetzten Chemikalien wurden wie kommerziell erhältlich und ohne weitere Aufreinigung in die durchgeführten Experimente eingesetzt.

Nylon 6/6 Pellets wurden von der Sigma Aldrich Corporation bezogen. Das Molekulargewicht der Verbindung wurde mittels Gelpermeationschromatographie bestimmt und auf 1,66 105 g/mol beziffert. N-Methylacetamid und 1,3,5-Trioxan wurden in einer Reinheit von jeweils 99% von der Sigma Aldrich Corporation bezogen, Bismuth-(III)-trifluormethansulfonat und Paraformaldehvd wurden in einem Reinheitsgrad zur Synthese, von der Sigma Aldrich Corporation bezogen. Wasser wurde vollentionisiert eingesetzt.

### Erste Modellreaktion

In einem ersten Modellexperiment wurde die bei dem erfindungsgemäßen Verfahren stattfindende chemische Reaktion an einem nicht-polymeren Modellmolekül, das eine Struktureinheit mit einer Stickstoff-Carbonylkohlenstoff-Bindung umfasst, erprobt. Als Modellmolekül wurde N- Methylacetamid gewählt.

Für das Experiment wurde ein Äquivalent N-Methylacetamid mit einem Äquivalent Paraformaldehyd in Präsenz von Bismut-Triflat als katalytisch aktive Lewis-Säure umgesetzt. Die chemische Reaktion wurde bei einer Reaktionstemperatur von 60 °C ohne Lösungsmittel oder mit Chloroform als Lösungsmittel durchgeführt.

Ohne an die Theorie gebunden zu sein, vermuten die Erfinder, dass bei der ausgeführten chemischen Reaktion das Carbonylkohlenstoff-Atom des Paraformaldehyds mit dem Stickstoff-Atom des sekundären Amins des N-Methylacetamids in einer nucleophilen Additionsreaktion reagiert hat, wobei die Reaktionsedukte nahezu vollständig zu einem Halbaminal umgewandelt wurden. Dabei griff in einem ersten Reaktionsschritt das freie Elektronenpaar des Stickstoff-Atoms des sekundären Amins vom N-Methylacetamid nucleophil das positiv polarisierte Carbonylkohlenstoff-Atom vom Paraformaldehyd an. Der darauffolgende Übergangszustand umfasste ein Molekül mit positiv geladenem Stickstoff sowie ein Alkoholat-Anion. In einem zweiten Reaktionsschritt fand eine Tautomerisierung eines Wasserstoff-Atoms statt. Dabei deprotonierte das Alkoholat-Anion den formal quartären Stickstoff, wodurch sich eine HydroxyGruppe und ein tertiärer Stickstoff bildeten. Auf diese Weise entstand schließlich das Halbaminal.

Es wurde herausgefunden, dass bei der Umwandlungsreaktion von N-Methylacetamid und Paraformaldehyd zum Halbaminal die Rückreaktion zu den Reaktionsedukten bei höheren Reaktionstemperaturen bevorzugt stattfindet.

In einer Folgereaktion wurde das Halbaminal in Präsenz von Bismut-Triflat als katalytisch aktive Lewis-Säure bei einer Reaktionstemperatur von 130 °C weiter umgewandelt. Dabei sind durch konzertierte Umlagerung Essigsäure und Formaldehyd-Aldimin entstanden.

Die Ausbeute an Essigsäure wurde aus einem Kernmagnetresonanz-spektroskopischen Signal relativ zum Reaktionsedukt N-Methylacetamid ermittelt. Sie betrug etwa 25 %.

Zur Kontrolle der Modellreaktion wurden zwei Blindreaktionen durchgeführt:

### Vergleichsreaktion A

Zunächst wurde die Reaktion ohne Formaldehyd oder Paraformaldehyd durchgeführt. Das heißt, N-Methylacetamid wurde mit 5 mol% Bismut-Triflat bei einer Reaktionstemperatur von 130 °C über Nacht angesetzt. Das entstandene Reaktionsprodukt war ein klares Öl. Bei einer Umsetzung des Amids wäre eine trübe Mischung zu erwarten gewesen. Entsprechend hat eine Untersuchung des Reaktionsprodukts mittels Kernmagnetresonanz-Spektroskopie ohne vorherige Aufbereitung ergeben, dass nur N-Methylacetamid, aber kein Halbaminal und keine Essigsäure im Reaktionsprodukt zu finden waren.

### Vergleichsreaktion B

Für eine zweite Blindreaktion wurde die Reaktion ohne Katalysator, also ohne Lewis-Säure, durchgeführt. Dafür wurde N-Methylacetamid mit Paraformaldehyd bei einer Reaktionstemperatur von 130 °C über Nacht angesetzt. Dabei ist, wie aus der Literatur bekannt, etwa 50 % des N-Methylacetamids zu dem Halbaminal umgesetzt worden (vgl. Chem Ber. 1961, 1879). Eine Weiterreaktion, bei der das Halbaminal in Essigsäure umgewandelt wurde, hat ohne Präsenz der als Katalysator fungierenden Lewis-Säure nicht stattgefunden.

### Zweite Modellreaktion

In einem zweiten Modellexperiment wurde die chemische Reaktion der ersten Modellreaktion näher untersucht.

Dafür wurde auf einer 3 mmol-Skala ein Äquivalent N-Methylacetamid mit einem Äquivalent Paraformaldehyd in Präsenz von 5 mol% Lewis-Säure umgesetzt. Die Reaktion wurde in einem 10 ml-Druckbehälter ohne Lösungsmittel bei 130 °C Reaktionstemperatur für 23,5 Stunden durchgeführt. Als Lewis-Säure wurde Scandium-Triflat, Neodym-Triflat, Samarium-Triflat, Yttrium-Triflat, Lanthan-Triflat, Bismut-Triflat oder Gadolinium-Triflat verwendet. Es wurde herausgefunden, dass sich bei der chemischen Reaktion Essigsäure und Methylamin bilden. Gemäß einer Kernmagnetresonanz-spektroskopischen Untersuchung in deuteriertem Wasser betrug die Ausbeute an Essigsäure bei der Verwendung von Bismut-Triflat als katalytische Lewis-Säure 19 %.

In einem weiteren Experiment wurde erneut auf einer 3 mmol-Skala ein Äquivalent N-Methylacetamid mit einem Äquivalent Paraformaldehyd in einem 10 ml-Druckbehälter in Präsenz von 5 mol% Bismut-Triflat als katalytisch agierende Lewis-Säure umgesetzt. Die Reaktion wurde ebenfalls ohne Lösungsmittel bei einer Reaktionstemperatur von 130 °C, diesmal für 19 Stunden durchgeführt. Dabei wurden entweder ein, zwei oder drei Äquivalente Wasser hinzugefügt. Das zugegebene Wasser bewirkte eine Verschiebung des Reaktionsgleichgewichts zugunsten der Reaktionsprodukte. Entsprechend hat sich in einer Kernmagnetresonanz-spektroskopischen Untersuchung des Reaktionsprodukts in Dimethylsulfoxid gezeigt, dass durch die Zugabe von drei Äquivalenten Wasser die Ausbeute an Essigsäure auf 22 % gesteigert werden konnte.

### Ausführunasbeispiel

In einem sich anschließenden Experiment wurde die erfindungsgemäße Reaktion dann auf ein Polymer angewendet.

Dafür wurden zunächst Polyamid 6.6 (226 mg, 1 mmol Wiederholungseinheiten, der vorgenannten Nylon 6/6 Pellets), Paraformaldehyd (60 mg, 2 mmol), 1,3,5-Trioxan (600 mg), Bismut-Triflat (33 mg, 0,05 mmol) und Wasser (54 µL, 3 mmol) in einem 10 ml-Druckbehälter gemischt. Dann wurde die Reaktionsmischung für 5 Stunden in einem temperierten Heizblock bei 165 °C gerührt. Im Anschluss wurde die Reaktionsmischung abgekühlt, in Wasser eingerührt und abfiltriert. Danach wurde der feste Filterrückstand mit kleinen Portionen Wasser gewaschen. Schließlich wurde der Filterrückstand für 30 Minuten bei einer Temperatur von 70 °C getrocknet. Auf diese Weise wurden 192 mg Reaktionsprodukt erhalten, was einer Reaktionsausbeute von 85 % entspricht.

Zur Quantifizierung der Depolymerisation des eingesetzten Polymers, das mit Paraformaldehyd als aktivierendem Reagenz und Bismut-Triflat als katalytisch aktiver Lewis-Säure gespalten wurde, wurden die Molekulargewichtsverteilung des Reaktionsedukts Polyamid 6.6 und die Molekulargewichtsverteilung des erhaltenen Reaktionsprodukts mittels ¹³C-Kernmagnetresonanz-Spektroskopie untersucht. Anhand der Verhältnisse charakteristischer Kohlenstoffsignale in der ¹³C-Kernmagnetresonanz-Spektroskopie kann die Molekulargewichtsverteilung bestimmt werden.

Für die ¹³C-Kernmagnetresonanz-Spektroskopie wurden die Proben in nichtdeuteriertem Trifluoroethanol gelöst. Die Messung hat beim Reaktionsedukt Polyamid 6.6 zu Signalen im Hochfeldbereich bei 25.4; 26.4; 28.9; 36.0; 40.1 ppm geführt. Beim Reaktionsprodukt wiederum sind im Hochfeldbereich Signale bei 24.4; 25.3; 33.7; 36.1; 43.9 ppm entstanden. Die Identifizierung und Zuordnung der Kohlenstoffsignale zu den entsprechenden Methylen-Gruppen der endständigen Monomere erfolgte über Messungen eigens synthetisierter oligomerer Hexamethylendiaminadipinsäureamide. Die Signalverhältnisse der intakten Polyamid 6.6-Polymerkette zu den neu gebildeten, endständigen Monomer-Gruppen stand im Verhältnis 1:0,31. Dadurch konnte auf einen Umsatz der im Polymer enthaltenen Amid-Gruppen von 24 % geschlossen werden, was eine Verkleinerung der durchschnittlichen Polymerkettenlängen um den Faktor 25 bedeutet.

## Patentansprüche

1. Verfahren zur Herstellung von Monomeren und/oder Oligomeren aus einem eine Struktureinheit mit einer Stickstoff-Carbonylkohlenstoff-Bindung umfassenden Polymer, wobei die Stickstoff-Carbonylkohlenstoff-Bindung in einer chemischen Reaktion mit Formaldehyd oder Paraformaldehyd als aktivierendes Reagenz gespalten wird und wobei die Reaktion unter Einsatz einer als Katalysator agierenden Lewis-Säure erfolgt.

2. Verfahren nach Anspruch 1, wobei Formaldehyd oder Paraformaldehyd stöchiometrisch oder substöchiometrisch eingesetzt wird.

3. Verfahren nach Anspruch 1 oder 2, wobei als Lewis-Säure ein Metallkatalysator eingesetzt wird.

4. Verfahren nach Anspruch 3, wobei der Metallkatalysator ein Metall umfasst aus der Gruppe: Aluminium, Bohr, Bismut, Cer, Eisen, Kupfer, Lanthan, Magnesium, Zinn, Titan, Zirkonium oder Zink.

5. Verfahren nach Anspruch 3 oder 4, wobei als Metallkatalysator eine Metall-Triflat-Verbindung, vorzugsweise Bismut-Triflat, eingesetzt wird.

6. Verfahren nach einem der Ansprüche 1 bis 5, wobei die chemische Reaktion in einem organischen Lösungsmittel, insbesondere in einem polaren organischen Lösungsmittel, durchgeführt wird.

7. Verfahren nach einem der Ansprüche 1 bis 6, wobei Wasser als Additiv eingesetzt wird.

8. Verfahren nach einem der Ansprüche 1 bis 7, wobei die Herstellung der Monomere und/oder Oligomere in einer eine erste und eine zweite Reaktionsstufe umfassenden Reaktion erfolgt.

9. Verfahren nach Anspruch 8, wobei die erste Reaktionsstufe bei einer Reaktionstemperatur von ≤ 100 °C, bevorzugt ≤ 80 °C, besonders bevorzugt ≤ 60 °C erfolgt.

10. Verfahren nach Anspruch 8, wobei die zweite Reaktionsstufe bei einer Reaktionstemperatur von ≤ 200 °C, bevorzugt ≤ 180 °C, besonders bevorzugt ≤ 165 °C erfolgt.

11. Verfahren nach Anspruch 8, wobei die erste und die zweite Reaktionsstufe bei einer Reaktionstemperatur von ≤ 200 °C, bevorzugt ≤ 180 °C, besonders bevorzugt ≤ 165 °C erfolgen.

12. Verfahren nach einem der Ansprüche 8 bis 11, wobei in der ersten Reaktionsstufe ein Halbaminal gebildet wird.

13. Verfahren nach einem der Ansprüche 8 bis 12, wobei in der zweiten Reaktionsstufe eine Carbonsäure und ein Aldimin gebildet werden.

14. Verfahren nach Anspruch 13, wobei das Aldimin weiter umgesetzt wird zu einem Amin.

15. Verfahren nach Anspruch 14, wobei das Amin ein Amin umfasst aus der Gruppe: Pentamethylendiamin (PDA), Hexamethylendiamin (HDA), Isophorondiamin (IPDA), 2,4'- und 4,4'-Diaminodicyclohexylmethan, 1,3- und 1,4-Diaminocyclohexan, 1,3- und 1,4-Bis-(aminomethyl)-cyclohexan (H6-XDA), 1,3- und 1,4-Bis(aminomethyl)benzol (XDA) oder Bis(aminomethyl)norbornan (NBDA).

16. Monomere, insbesondere ein Carbonsäure-Monomer und ein Aldimin-Monomer oder ein Amin-Monomer, erhältlich oder hergestellt aus einem Verfahren nach einem der Ansprüche 1 bis 15.
